# EUROPEAN PATENT APPLICATION

(11) **EP 4 249 914 A1**
(43) Date of publication of application: **27.09.2023**
(21) Application number: 21894070.8
(22) Date of filing: 22.11.2021
(51) Int. Cl.: G01N 33/53

(54) **APPARATUS AND METHOD FOR DETECTING MISFOLDED PROTEIN IN BIOLOGICAL SAMPLE**

(30) Priority: 21.11.2020 CN 202011315496; 15.11.2021 CN 202111346532
(71) Applicant: Shuwen Biotech Co., Ltd., Huzhou, Zhejiang 313200 (CN)
(72) Inventor: LI, Xingmin, Huzhou, Zhejiang 313200 (CN)
(74) Representative: HGF
(86) International application number: PCT/CN2021/132194
(87) International publication number: WO 2022/105923

(57) **Abstract**

An apparatus and method for detecting a misfolded protein in a biological sample. The method comprises: (a) providing a biological sample; (b) mixing the biological sample with a detection reagent, the detection reagent being capable of binding to a misfolded protein; (c) enabling the biological sample mixed with the detection reagent to come into contact with and pass through a separation matrix, the separation matrix being constructed to adsorb the detection reagent that does not bind to the misfolded protein and to allow passage of the detection reagent binding to the misfolded protein; (d) collecting, in a free liquid state, the biological sample passing through the separation matrix; and (e) detecting the presence of the detection reagent in the collected biological sample in the free liquid state, wherein the presence of the detection reagent indicates the presence of a misfolded protein in the biological sample. The apparatus and method can be used for diagnosing and predicting diseases characterized by a misfolded protein, e.g. can be used for diagnosing whether a pregnant woman suffers from preeclampsia or is at risk of suffering from preeclampsia.

## Description

### Technical Field

The present application relates to biological detection technology, and more particularly, to an apparatus and a method for detecting a misfolded protein in a biological sample.

### Background Art

Preeclampsia is a disease related to the pregnancy process. At present, little is known about its pathogenesis. Preeclampsia usually refers to the occurrence of hypertension and proteinuria after 20 weeks of pregnancy in pregnant women with normal blood pressure before pregnancy, accompanied by headache, dizziness, nausea, vomiting, epigastric discomfort and other symptoms.

At present, the clinical diagnosis of preeclampsia mainly depends on its clinical features such as hypertension and proteinuria. Researchers have been working on new markers and new methods for the diagnosis of preeclampsia. Researchers from Yale University in the United States find that some misfolded protein molecules in the urine of pregnant women are significantly related to the occurrence of preeclampsia. These misfolded protein molecules can selectively bind to Congo red dye, and Congo red dye can also bind to cellulose, and thus a spot-diffusion based assay is developed for the detection of misfolded proteins in the urine of pregnant women for the diagnosis or prediction of preeclampsia.

However, the spot diffusion technique adopted in the prior art is not suitable for processing weak positive samples, and it is difficult to effectively distinguish weak positive samples from negative samples, which may easily lead to misjudgment. Therefore, it is necessary to provide an improved detection method and apparatus. Additionally, there is a need in the art for detection methods and apparatuses that enable quantification and/or have improved sensitivity, specificity and accuracy.

### Summary of the Invention

The present disclosure satisfies at least one of the above needs. One object of the present application is to provide an apparatus and a method capable of effectively detecting a misfolded protein.

In one aspect of the present application, a method for detecting a misfolded protein or an aggregate thereof in a biological sample is provided and comprises: (a) providing a biological sample; (b) mixing the biological sample with a detection reagent, the detection reagent being capable of binding to the misfolded protein or the aggregate thereof; (c) enabling the biological sample mixed with the detection reagent to come into contact with and pass through a separation matrix, the separation matrix being constructed to adsorb the detection reagent that does not bind to the misfolded protein or the aggregate thereof and to allow passage of the detection reagent binding to the misfolded protein or the aggregate thereof; (d) collecting, in a free liquid state, the biological sample passing through the separation matrix; and (e) detecting the presence of the detection reagent in the collected biological sample in the free liquid state, wherein the presence of the detection reagent indicates the presence of the misfolded protein or the aggregate thereof in the biological sample. In one embodiment, in step (d), the biological sample is collected in a fluid collection chamber, and the collected biological sample is in a free liquid state in the fluid collection chamber. In a further embodiment, the collected biological sample can freely flow in the fluid collection chamber. In a still further embodiment, the volume of freely flowing liquid in the fluid collection chamber is at least about 50 µL, at least about 100 µL, at least about 500 µL, at least about 50 µL, at least about 1 mL, at least about 3 mL, or about 3 mL to about 5 mL.

In another aspect of the present application, an apparatus for detecting a misfolded protein or an aggregate thereof in a biological sample is provided and comprises: a housing defining a fluid separation chamber and a fluid collection chamber, wherein the fluid separation chamber is used to receive the biological sample mixed with a detection reagent, wherein the detection reagent is capable of binding to the misfolded protein or the aggregate thereof in the biological sample, and the fluid separation chamber is also for separating and retaining the detection reagent that does not bind to the misfolded protein or the aggregate thereof in the biological sample from the biological sample mixed with the detection reagent, and allows the detection reagent that binds to the misfolded protein or the aggregate thereof in the biological sample to flow through; and wherein the fluid collection chamber is used to collect the biological sample flowing through the fluid separation chamber.

In another aspect of the present application, a kit is also provided and the kit comprises the apparatus for detecting a misfolded protein in a biological sample according to the aforementioned aspects.

In yet another aspect of the present application, the use of the apparatus of the present disclosure in the manufacture of a kit for diagnosing or predicting a disease characterized by a misfolded protein is provided.

In yet another aspect of the present application, a method of predicting the risk of suffering from a disease characterized by a misfolded protein is provided.

In yet another aspect of the present application, the use of a combination of (1) a detection reagent capable of specifically binding to a misfolded protein or an aggregate thereof and (2) an adsorption medium capable of adsorbing the detection reagent in a free state in the preparation of an apparatus or kit for diagnosing or predicting a disease characterized by the misfolded protein is provided.

The above is an overview of the application, and there may be simplifications, generalizations, and omissions of details, so those skilled in the art should recognize that this section is illustrative only and is not intended to limit the scope of the application in any way. This Summary section is not intended to identify key features or essential features of the claimed subject matter, nor is it intended to be used as an aid in determining the scope of the claimed subject matter.

### Brief Description of the Drawings

The foregoing and other features of the present application will be more fully and clearly understood from the following specification and appended claims in conjunction with the accompanying drawings. It can be understood that these drawings only depict some implementations of the content of the application, and therefore should not be considered as limiting the scope of the content of the application. By using the accompanying drawings, the content of the application will be explained more clearly and in detail.
Fig. 1 shows an apparatus 100 for detecting a misfolded protein in a biological sample according to one example of the present application;
Fig. 2 shows an apparatus 200 for detecting a misfolded protein in a biological sample according to one example of the present application;
Fig. 3a-3b show an apparatus 300 for detecting a misfolded protein in a biological sample according to one example of the present application;
Fig. 4a-4c show an apparatus 400 for detecting a misfolded protein in a biological sample according to one example of the present application;
Fig. 5 shows a method 500 for detecting a misfolded protein in a biological sample according to one example of the present application.
Fig. 6 shows the detection results of simulated samples. The left side shows the collected liquid of a simulated positive sample; and the right side shows the collected liquid of a simulated negative sample.
Fig. 7 shows the results of the alumina filtration urine color test.
Fig. 8 shows detection sensitivity tests of alumina as a separation material.
Fig. 9a-9c show the results of detecting simulated samples using cellulose acetate (Fig. 9a), dry protein (Fig. 9b) and microcrystalline cellulose (Fig. 9c) as separation media, respectively. The left side in each figure is the collected liquid of a simulated negative sample; the right side is the collected liquid of a simulated positive sample.
Fig. 10a-10c show the process of clinical detection using the detection apparatus of the present invention.

### Detailed Description of Embodiments

The terms "substantially", "about" and similar terms, as used herein, are used as terms of approximation rather than terms of degree, and are intended to explain the inherent deviation in the measured or calculated values recognized by those skilled in the art. Likewise, it is intended that any numerical range recited herein comprises all subranges subsumed within the stated range to the same numerical precision. For example, the range "1.0-10.0" is intended to include all subranges between (or comprising) the minimum value of 1.0 and the maximum value of 10.0, *i.e.,* having a minimum value greater than or equal to 1.0 and a maximum value less than or equal to 10.0, for example, 2.4-7.6. It is intended that any maximum numerical limitation stated herein comprises all lower numerical limitations contained therein, and it is intended that any minimum numerical limitation stated in this specification comprises all larger numerical limitations contained therein. Accordingly, the inventors reserve the right to modify this specification, comprising the claims, to expressly describe any subranges subsumed within the range expressly described herein. As used herein, the term "about" refers to a value that is within 10% above or below the stated value.

In the following detailed description, reference is made to the accompanying drawings which form a part of the present application. In the drawings, similar symbols typically represent similar components, unless context dictates otherwise. The illustrative embodiments described in the detailed description, drawings, and claims are not intended to be limiting. Other embodiments may be utilized and other changes may be made without departing from the spirit or scope of the subject matter of the present application. It can be understood that various aspects of the content of this application, which are generally described in this application and illustrated in the attached drawings, can be configured, substituted, combined and designed in many different forms, and all of these clearly constitute a part of this application.

The researchers found unique nonrandom cleavage products of SERPINA-1 and albumin in urine samples from women with preeclampsia. This propensity of SERPINA-1 fragments to misfold and form supramolecular aggregates has led to consideration of detection of misfolded proteins for early diagnosis of preeclampsia. Further studies have found that misfolded proteins present in the urine of women with preeclampsia can bind to Congo red dye, so the prior art uses test papers loaded with Congo red dye to detect misfolded proteins indicating preeclampsia. For example, Chinese patent application CN 108291905 A adopts lateral flow chromatography technology, and Congo red dye is embedded in a sample pad in a freeze-dried form. After the urine sample is added, the urine is mixed with Congo red dye and passed through a capturer, where free Congo red dye is captured by the capturer and Congo red dye that binds to the protein of interest (*i.e.,* a misfolded protein) in the urine flows through the capturer to enter the display strip zone. Thus, if the protein of interest is present in the urine, it will appear red (positive) in the display strip zone, and if the protein of interest is absent in the urine, the free Congo red dye will be captured by the capturer, thus showing colorless (negative) in the display strip zone. Similarly, the Chinese patent application CN 109342737 A adopts the spot diffusion technology on the test papers, that is, after the urine sample is mixed with Congo red dye, a certain amount of urine is spotted onto the filter paper through a capillary tube. For a negative sample, the Congo red dye in the negative sample binds to the filter paper to form a concentrated red spot, while for a positive sample, because Congo red dye binds to a target protein, the lack of free Congo red dye in the sample leads to the failure to form a concentrated red spot on the filter paper, instead, a red circle with uniform diffusion forms, that is, spot diffusion. Early diagnosis of preeclampsia can be made by determining whether a concentrated red spot or a diffuse spot is formed.

However, the inventors of the present application find that the existing test papers for detecting misfolded proteins have defects, which are not suitable for processing weak positive samples. When weakly positive samples are detected with test papers, since the amount of misfolded proteins contained in weakly positive samples is relatively small, some Congo red dye may not bind to misfolded proteins, which makes a certain amount of free Congo red dye still exist in test papers. Excessive free Congo red dye can also form a concentrated red spot, or develop color in the display strip zone, leading to false diagnosis results. In addition, for lateral flow chromatography, only semi-quantitative results can be obtained at best, and the detection specificity and accuracy are not ideal due to the limited sample volume that can be added (µL level).

In order to solve one or more of the above-mentioned problems existing in the prior art, the inventors of the present application have abandoned the traditional test papers or the lateral flow technology based on capillary action or absorbent pad suction, but creatively detect misfolded proteins in a liquid state. Specifically, the inventors used a container to load a solution of a biological sample mixed with a dye such as Congo red dye, and the color of the collected solution after the mixed liquid was treated with a separation matrix can be observed to indicate whether there is a misfolded protein in the biological sample. The darker the color of the collected solution, the lower the proportion of the dye adsorbed by the separation matrix, and the higher the proportion of the dye that binds to a misfolded protein. The liquid-based detection method and apparatus of the present disclosure can not only perform qualitative or semi-quantitative detection through visual inspection, but also allow quantitative detection through optical detection equipment. Importantly, the inventors of the present application have also unexpectedly found that the liquid-based detection method and apparatus of the present disclosure also achieve significantly improved specificity and accuracy.

Based at least in part on the above findings, the present disclosure provides a liquid-based detection technique.

In one aspect, a method for detecting a misfolded protein or an aggregate thereof in a biological sample is provided and comprises: (a) providing a biological sample; (b) mixing the biological sample with a detection reagent, the detection reagent being capable of binding to the misfolded protein or the aggregate thereof; (c) enabling the biological sample mixed with the detection reagent to come into contact with and pass through a separation matrix, the separation matrix being constructed to adsorb the detection reagent that does not bind to the misfolded protein or the aggregate thereof and to allow passage of the detection reagent binding to the misfolded protein or the aggregate thereof; (d) collecting, in a free liquid state, the biological sample passing through the separation matrix; and (e) detecting the presence of the detection reagent in the collected biological sample in the free liquid state, wherein the presence of the detection reagent indicates the presence of the misfolded protein or the aggregate thereof in the biological sample. In one embodiment, in step (d), the biological sample is collected in a fluid collection chamber, and the collected biological sample is in a free liquid state in the fluid collection chamber.

In some embodiments, the biological sample collected in a free liquid state is not substantially attached or adsorbed or bound to the inner wall of the fluid collection chamber, but is able to flow freely within the fluid collection chamber. This is different from lateral flow chromatography, wherein the sample flow is directed from the sample loading side to the detection side based on capillary action (capillary bed) or absorbent pad suction, and the sample in the detection zone is constrained or limited by capillary action or absorption pad suction. The limitation of capillary action or absorption pad suction also limits the volume of sample addition allowed by lateral flow chromatography. The use of the capillary bed or absorbent pad also makes it impossible to carry out quantitative detection by an optical method.

In some embodiments, the collected biological sample can freely flow in the fluid collection chamber. In some embodiments, the volume of freely flowing liquid in the fluid collection chamber is at least about 50 µL, at least about 100 µL, at least about 500 µL, at least about 50 µL, at least about 1 mL, at least about 3 mL, or about 3 mL to about 5 mL, for example about 0.6 mL, .7 mL, 0.8 mL, 0.9 mL, 1 mL, 1.1 mL, 1.2 mL, 1.3 mL, 1.4 mL, 1.5 mL, 1.6 mL, 1.7 mL, 1.8 mL, 1.9 mL, 2.0 mL, 2.1 mL, 2.2 mL, 2.3 mL, 2.4 mL, 2.5 mL, 2.6 mL, 2.7 mL, 2.8 mL, 2.9 mL, 3.0 mL, 3.5 mL, 4.0 mL, 4.5 mL, 5.0 mL, 6.0 mL, 7.0 mL, 8.0 mL, 9.0 mL, or 10 mL.

In some embodiments, the liquid-based detection technique of the present disclosure allow for the addition of a biological sample having a volume of at least about 50 µL, at least about 100 µL, at least about 0.5 mL, at least about 1 mL, at least about 3 mL, or about 3 mL to about 5 mL, for example about 0.6 mL, 0.7 mL, 0.8 mL, 0.9 mL, 1 mL, 1.1 mL, 1.2 mL, 1.3 mL, 1.4 mL, 1.5 mL, 1.6 mL, 1.7 mL, 1.8 mL, 1.9 mL, 2.0 mL, 2.1 mL, 2.2 mL, 2.3 mL, 2.4 mL, 2.5 mL, 2.6 mL, 2.7 mL, 2.8 mL, 2.9 mL, 3.0 mL, 3.5 mL, 4.0 mL, 4.5 mL, 5.0 mL, 6.0 mL, 7.0 mL, 8.0 mL, 9.0 mL, or 10 mL.

In some embodiments, the liquid-based detection technique of the present disclosure allows for quantitative detection of the collected liquid by an optical method. In some embodiments, at least a portion of the fluid collector is transparent (for example, providing a transparent viewing window) for a user to observe the color of the mixed solution or detect the mixed solution by an optical method. The optical method that can be used in the present disclosure is known in the art of optical analysis, comprising but not limited to spectrophotometry. The optical method that can be used in the present invention can also be selected according to the detection reagent employed, for example, when the detection reagent is a fluorescent dye, then fluorescence analysis can be used.

In one aspect, the present disclosure provides an apparatus for detecting a misfolded protein or an aggregate thereof in a biological sample, and the apparatus comprises: a housing defining a fluid separation chamber and a fluid collection chamber, wherein the fluid separation chamber is used to receive a mixed liquid of the biological sample and a detection reagent, wherein the detection reagent is capable of binding to the misfolded protein or the aggregate thereof in the biological sample, the fluid separation chamber contains a separation matrix constructed to adsorb the detection reagent that does not bind to the misfolded protein or the aggregate thereof from the mixed liquid and to allow passage of the detection reagent binding to the misfolded protein or the aggregate thereof; and the fluid collection chamber is used to collect the biological sample flowing through the separation matrix in the fluid separation chamber.

In some embodiments, the detection reagent such as Congo red dye can be preloaded in the container prior to the detection process, or can also be added to the container during the detection process (in a solid or solution form). In other embodiments, the biological sample can also be mixed with the detection reagent, such as Congo red dye, in the same container, or can be pre-mixed with the detection reagent, such as Congo red dye, in other containers.

As used herein, "container" or "housing" refers to an element that can be used to hold a liquid and preserve or collect a liquid such as a biological sample in a liquid form, and may have, for example, a structure similar to a graduated cylinder, a beaker, a bottle, a test tube, or any other chambers that can hold liquids. The configuration of the chamber comprises, but is not limited to, cylindrical shape, cuboid shape, conical shape, hemispherical shape, or a combination thereof. The container or housing may comprise one or more chambers for different processing operations or steps, respectively, in the detection process, wherein each chamber may be used for one or more operations or steps. It can be understood that the container or housing may generally be formed from a colorless transparent material, such as glass, acrylic, plastic, polyester or other materials. The polyester may be, for example, polyvinyl chloride. In some embodiments, a portion of the container may be formed from an opaque material, but at least a portion of the area of the container is transparent (for example, providing some transparent viewing windows) for a user to observe the color of the mixed solution or detect the mixed solution by an optical method.

The container or housing may comprise one or more chambers, for example, comprising one or more of a "fluid receiving chamber", a "fluid separation chamber" and a "fluid collection chamber". At least some of the chambers are in fluid communication with each other or can otherwise transfer liquid to each other, such as by pouring or injecting liquid from one chamber into another chamber. Depending on the specific process, the chamber may or may not contain a substance. In some embodiments, the chamber may contain a detection reagent, such as a dye, capable of binding to a misfolded protein or other proteins of interest in a biological sample, or contain a separation matrix material capable of capturing or adsorbing a dye. In some embodiments, the detection reagent can be, for example, a dye, such as a visible light dye or a fluorescent dye, such as an azo dye or an analog thereof (such as Congo red or Evans blue), a benzothiazole dye or an analog thereof (such as Thioflavin T and Thioflavin S), Amaranth red, Brilliant black, Nile red, or a combination of one or more thereof. In some embodiments, the apparatus and method of the present application are used for early detection of preeclampsia, and accordingly, the detection reagent that can be used for binding to a misfolded protein of preeclampsia may be an azo dye or an analog thereof, such as Congo red dye (i.e., 4-amino-3-[4-[4(1-amino-4-sulfo-naphthalene-2-yl)azophenyl]phenyl]azo-naphthalene-1-sulfonic acid disodium). In some embodiments, the detection reagent may be contained in the chamber in a solid form, or may be contained in the chamber in a solution form. Preferably, the detection reagent can be stored in a solid form, so that when added to a biological sample, the detection reagent is mixed with the biological sample and exists in a liquid form, which allows the detection reagent to sufficiently bind to a protein of interest. The detection reagent stored in a non-solution form has long-term stability or a long shelf life. In one embodiment, the dry detection reagent is Congo red, and can be stored in the chamber in an amount of, for example, 0.1 µg-100 µg, more preferably 0.2 µg-50 µg, even more preferably 1 µg to 25 µg (for example, 2 µg, 5 µg, 10 µg, 15 µg or 20 µg). Quantities are kept in the chambers, or stored in a sealed bag and added to a corresponding chamber before the detection or during the detection. The color of the detection reagent is detectable, *i.e.,* visible to the naked eye, and can be detected by visual inspection and/or mechanical or electronic readers.

As previously mentioned, some of the chambers may contain a separation matrix material. The separation matrix material can compete with a protein of interest or some other specific proteins for binding the detection reagent due to the special spatial structure and/or composition of the separation matrix material. It is worth noting that the detection reagent has a lower affinity for the separation matrix material than for the protein of interest (a misfolded protein or an aggregate thereof). That is, if the protein of interest is not present in the sample, the detection reagent will bind to the separation matrix material, so that the detection reagent cannot remain in the collected biological sample and allow it to develop color; However, if the protein of interest is present in the biological sample, the detection reagent will bind to the protein of interest and not bind to the separation matrix material, so the binding product of the protein of interest and the detection reagent will remain in the liquid and flow into the fluid collection chamber, thereby forming an observable colored solution. In some embodiments, the separation matrix material can be any material (for example, containing free hydroxyl groups) known to those skilled in the art that can bind to a free detection reagent through hydrogen bonds or van der Waals forces, *etc.,* or a medium with a microporous structure on the surface, such as an adsorption medium or a molecular sieve chromatography medium. In some embodiments, the separation matrix does not adsorb or bind to the protein of interest (*i.e.,* a misfolded protein or an aggregate thereof). In some embodiments, the separation matrix can be a particulate (for example, particle size distribution in the range of about 100-200 mesh, for example, about 110 mesh, about 120 mesh, about 130 mesh, about 140 mesh, about 150 mesh, about 170 mesh, about 180 mesh or about 190 mesh), a fiber, or a semi-solid (for example, gel) form.

the separation matrix can be selected based on the affinity of the detection reagent employed and the protein of interest. In some embodiments, the material of the separation matrix may comprises, but is not limited to: cotton (such as cotton or cotton fibers), or cotton gauze; silk; a cellulose, such as nitrocellulose, microcrystalline cellulose, cellulose acetate, or wood chips; a polymer, such as polyester, polyethylene, polysulfone, polyvinyl alcohol, polyethylene glycol (such as PEG2000, PEG3000, PEG4000, PEG5000 or PEG6000), or polyacrylamide; glass fiber; silica gel, gelatin or dextran gel; a dry protein or a protein dry powder, such as egg white protein dry powder; an inorganic mineral soil, such as zeolite, clay, kaolin, hydroxyapatite and montmorillonite; a calcium salt, such as calcium chloride, calcium carbonate or calcium phosphate; activated carbon; or activated alumina; or any combination of the above. In some embodiments, the material of the separation matrix is selected from activated alumina, microcrystalline cellulose, cellulose acetate, dry protein, or any combination thereof. In some embodiments, the dry protein or protein dry powder may have a particle size of about 0.5-1 mm or greater, and/or may contain a Congo red-tropic protein or have a beta sheet structure. In one embodiment, the activated alumina is acidic alumina and may have a particle size of about 100-200 mesh, such as about 100-150 mesh.

In some embodiments, the fluid chambers of the housing can be in fluid communication with each other. Preferably, these fluid chambers may be separated by an isolation layer having micropores or other similar structures, such as a sieve plate, a partition plate or a woven net. The isolation layer prevents the solid material (for example, the separation matrix material) filled in one fluid chamber from transferring to the other fluid chamber, but still allows liquid to flow between the fluid chambers. In some embodiments, the isolation layer can hold the solid material in place. In one embodiment, the apparatus of the present disclosure further comprises an isolation layer disposed between the fluid separation chamber and the fluid collection chamber and separating the fluid separation chamber and the fluid collection chamber. The isolation layer allows the biological sample to flow through and prevents the separation matrix from entering the fluid collection chamber. The isolation layer may be in the form of a sieve plate, a partition plate or a woven net and may be one or more layers.

In some embodiments, the material of the isolation layer does not adsorb biomacromolecules, such as proteins. In some embodiments, the material of the isolation layer having micropores, such as a sieve plate, a partition plate or a woven net, can comprise an inorganic material (such as inorganic fibers or particulates), such as ceramics, glass, glass fiber, or metals (such as stainless steel); a polymer, comprising a polymer fiber or a polymer particulate, such as a polyamide (such as nylon), a polyethylene (such as ultra-high molecular weight polyethylene (UHMW-PE), polytetrafluoroethylene (PTFE), polystyrene, or polyvinyl chloride (PVC)), a polyacrylic (such as acrylic), a polypropylene, a plastic (such as a porous plastic), a polyester and a polyurethane; cellulose, such as filter paper or wood pulp cellulose; or other materials that allow the passage of the biological sample and prevent the separation matrix from entering the fluid collection chamber.

In some embodiments, the material of the isolation layer is porous plastic. The porous plastic, also known as foamed plastic, is a substance with fine pore spongy structure formed by taking a synthetic resin as matrix and adding a foaming agent and other additives through foaming. Commonly used resins comprise polyethylene, polystyrene, polyvinyl chloride, polypropylene, polyurethane, *etc.*

In some embodiments, the material of the isolation layer is hydrophilic or hydrophobic. Hydrophobic materials can be made hydrophilic by surface modification with hydrophilic agents or by plasma treatment.

In some embodiments, the isolation layer can be one or more layers. In some embodiments, the isolation layer may have a thickness of about 0.1-5 mm, such as about 0.5-4 mm, about 1-3 mm, about 1.5-2.5 mm, about 1.2 mm, about 1.3 mm, about 1.4 mm, about 1.5 mm, about 1.6 mm, about 1.7 mm, about 1.8 mm, about 1.9 mm, about 2.0 mm, about 2.3 mm and about 3.2 mm.

Depending on the size of the separation matrix employed, the separation layer material may have, for example, a pore size of about 5 µm-200 µm, such as 5 µm, 10 µm, 20 µm, 30 µm, 40 µm, 50 µm, 60 µm, 70 µm, 80 µm, 90 µm, 100 µm, 110 µm, 120 µm, 130 µm, 140 µm, 150 µm, 160 µm, 170 µm, 180 µm or 190 µm. The pore size of the isolation layer material can also be smaller than 5 µm or larger than 200 µm, as long as it can prevent the separation matrix from entering the fluid collection chamber.

In an additional embodiment, the present disclosure provides a porous isolation element constructed to allow passage of the biological sample and prevent entry of the separation matrix. In some embodiments, the porous isolation element is a sieve plate. In some embodiments, the porous isolation element is prepared from a material selected from: inorganic materials (such as inorganic fibers or particulates), such as ceramics, glass, glass fiber, or metals (such as stainless steel); a polymer, comprising a polymer fiber or a polymer particulate, such as a polyamide (such as nylon), a polyethylene (such as ultra-high molecular weight polyethylene (UHMW-PE), polytetrafluoroethylene (PTFE), polystyrene, or polyvinyl chloride (PVC)), a polyacrylic (such as acrylic), a polypropylene, a plastic (such as a porous plastic), a polyester and a polyurethane; cellulose, such as filter paper or wood pulp cellulose; or any combinations thereof. In some embodiments, the porous isolation element may have a thickness of about 0.1-5 mm, such as about 0.5-4 mm, about 1-3 mm, about 1.5-2.5 mm, about 1.2 mm, about 1.3 mm, about 1.4 mm, about 1.5 mm, about 1.6 mm, about 1.7 mm, about 1.8 mm, about 1.9 mm, about 2.0 mm, about 2.3 mm and about 3.2 mm. In some embodiments, the porous isolation element may have, for example, a pore size of about 5 µm-200 µm, such as 5 µm, 10 µm, 20 µm, 30 µm, 40 µm, 50 µm, 60 µm, 70 µm, 80 µm, 90 µm, 100 µm, 110 µm, 120 µm, 130 µm, 140 µm, 150 µm, 160 µm, 170 µm, 180 µm or 190 µm.

In the present application, the protein of interest capable of binding to the detection reagent in the biological sample may be, for example, a misfolded protein or an aggregate thereof or a supramolecular protein aggregate, or a mixture of the aforementioned substances and a fragment of each protein. The protein of interest may contain a beta sheet structure. In addition, the protein of interest can be Congo red dye-tropic. The misfolded protein can comprise, for example, alpha-1 antitrypsin (SerpinAl), ceruloplasmin, heavy chain IgG, light chain IgG, interferon induced protein 6-16 (IF 16-6, G1P3), albumin, a mixture thereof or a fragment thereof or a fragment of each protein. However, the misfolded protein is not limited to the aforementioned proteins. For example, the misfolded protein can be any protein that causes or is associated with a protein misfolding disorder, or a combination the proteins.

The detection method and apparatus of the present application can be used to detect a misfolded protein, an aggregated protein and/or a supramolecular aggregated protein in a biological sample, wherein the biological sample is from mammals, comprising but not limited to human, primates or genetically engineered mammals, and the mammals may be pregnant. The detection method, apparatus and kit herein can be used to indicate or predict the risk of a subject suffering from eclampsia or preeclampsia.

The biological sample may be a fluid such as urine, blood, saliva, tissue/interstitial fluid, serum, plasma, cerebrospinal fluid, and amniotic fluid. In other embodiments, the biological sample may be solid and processed into a liquid prior to detection. The detection method of the present application can be used on biological samples from mammals. In the case of human, the method may be used for pregnant women who have a pregnancy (*i.e.,* gestational age) of about 8 to 42 weeks, preferably about 18 to 41 weeks, more preferably about 20 to 41 weeks or 20 weeks to childbirth. However, the detection method of the present application can also be used in postpartum mammals. At least one protein of interest detected by the liquid-based detection apparatus and method of the present disclosure can be detected visually to obtain qualitative or semi-quantitative results or optically to obtain qualitative results. For example, the darker the color of the biological sample separated by the separation matrix and collected, the higher the content of the protein of interest in the biological sample, and the greater the possibility that the tested patient suffers from related disorders.

The detection apparatus of the present application can also be used to detect protein misfolding disorders other than eclampsia and preeclampsia (comprising severe preeclampsia and atypical preeclampsia). For example, the apparatus can be used to detect misfolded proteins in the following misfolded protein disorders or conditions, such as Alzheimer's disease, cerebral beta-amyloid angiopathy, retinal ganglion cell degeneration in glaucoma, prion disease, Parkinson's disease and other synucleinopathy, tauopathies, frontotemporal lobar degeneration (FTLD), FLTD_FUS, amyotrophic lateralizing sclerosis (ALS), Huntington's disease and other trilogy, repetition disorder, dementia (UK and Danish familial), hereditary cerebral hemorrhage with amyloidosis, CADASIL, Alexander disease, various amyloidosis, Serinopathies, Type II diabetes, inclusion body myositis/myopathy, cataract, Retinitis pigmentosa with rhodopsin mutation, medullary thyroid carcinoma, pituitary prolactinoma, hereditary lens corneal dystrophy, Mallory body, pulmonary alveolar proteinosis, odontogenic neoplastic amyloidosis, cystic fibrosis, sickle cell disease and critical illness myopathy.

In some embodiments, the detection apparatus and method of the present application can provide test results in about 10 minutes or less, in about 5 minutes or less, preferably in about 3 minutes or less.

The present application also comprises a kit for detecting a protein of interest in a biological sample. The kit may comprise an apparatus for detecting a misfolded protein in a biological sample of the present disclosure, and/or other components required for detection. For example, the kit may also comprise an apparatus for adding a biological sample into a housing, such as a pipette or dropper, and instructions for use of the apparatus. In some embodiments, the kit may also comprise a color comparison table with different color depths, so that users can make determination based on the actual detection results (colors) against the table. The apparatus can be used not only as a stand-alone entity but also in the form of a kit, which may be advantageous in certain clinical or non-clinical settings. The kit can be packaged in a box or a polyester film bag. The kit can also be packaged individually or in multiples, for example, 2, 5, 10, 15, 25, 50 or 100 kits per package.

In some embodiments, the pipette or dropper is constructed as a fluid receiving chamber in which a detection reagent is contained. The detection reagent can be in a powder or particulate form. In this way, when the fluid receiving chamber receives the biological sample, the detection reagent stored therein can be mixed and dissolved into the biological sample as expected, so as to be able to sufficiently bind to the protein of interest in the biological sample. The detection reagent may also take other solid forms. For example, the fluid receiving chamber may have a matrix material therein (other than the separation matrix material described herein), such as a sponge or similar structure, and the detection reagent may be dispersedly attached in the matrix material. When the biological sample is added into the fluid receiving chamber, the detection reagent attached to the matrix material can be quickly dissolved into the biological sample. Alternatively, the detection reagent can also take a solution form, and when the fluid receiving chamber receives the biological sample, the solution of the detection reagent can also be quickly and sufficiently mixed with the biological sample. In some embodiments, a control line (for example, a positive control, such as a beta sheet) may also be provided within the pipette or dropper.

The detection method and apparatus of the present application may be used by medical personnel, non-professional medical personnel, or by the patient himself (when the patient is human) in a clinical laboratory (in a hospital or out-of-hospital environment), an immediate care environment, a physician's office laboratory, an emergency room (for example, in a hospital), or as a near-patient testing apparatus or a point-of-care apparatus. In addition, the detection method and apparatus of the present application can be combined with other diagnostic assays (such as immunoassays, such as those assays for detecting other proteins associated with preeclampsia (*i.e.,* biomarkers, such as sFlt-1, PLGF, PP-A, PP13, pentraxin, inhibin-A, and soluble endoglin)) and/or other methods or observation methods commonly used in the diagnosis of preeclampsia (such as blood pressure readings, clinical tests used in the diagnosis of preeclampsia, comprising platelet count, serum creatinine concentration, serum ALT (alanine aminotransferase) and AST (aspartate aminotransferase), and other signs or symptoms such as weight gain, dizziness, headache, blurred vision, *etc.*)*.* It can be understood that this application does not limit the use environment of the detection method and apparatus.

Next, the apparatus and method of the present application will be further described in conjunction with specific embodiments.

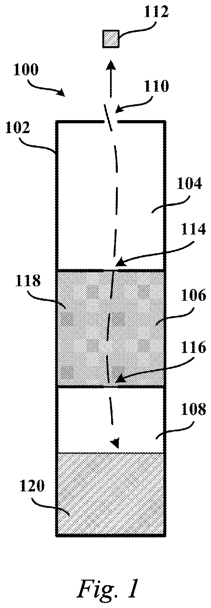

Fig. 1 shows an apparatus 100 for detecting a misfolded protein in a biological sample according to one embodiment of the present application.

As shown in Fig. 1, the apparatus 100 comprises a housing 102, and the housing 102 may comprise one or more fluid chambers. In the embodiment shown in Fig. 1, the housing 102 defines a fluid receiving chamber 104, a fluid separation chamber 106, and a fluid collection chamber 108. The fluid chambers 104, 106, and 108 may be in fluid communication with each other, allowing fluid to flow within the different fluid chambers in a predetermined sequence.

Specifically, the fluid receiving chamber 104 is located at the uppermost end of the housing 102, and it is operatively communicated with the outside through the adjacent fluid inlet 110 on the housing 102. In some embodiments, a removable sealing plug 112 is installed on the fluid inlet 110, and when the apparatus 100 is temporarily stored without use, installing the sealing plug 112 on the fluid inlet 110 may maintain the hermetic seal of the interior space of the housing 102 so as to avoid contamination of the interior space. When it is necessary to use the apparatus 100 to detect a biological sample, the sealing plug 112 can be removed from the fluid inlet 112, thereby exposing the interior of the fluid receiving chamber 104, so that the biological sample or other liquids can be injected into the fluid receiving chamber 104 through the fluid inlet 110. It is understood that in some embodiments, the fluid inlet 110 may also be sealed by other structures. For example, the fluid inlet 110 may be sealed by a pierceable or removable sealing membrane that may be pierced or removed prior to use of the apparatus 100 for detection to allow fluid injection.

In some embodiments, the fluid receiving chamber 104 contains a detection reagent. Preferably, the detection reagent may be in powder or particulate form. In this way, when the fluid receiving chamber 104 receives the biological sample, the detection reagent stored therein can be mixed and dissolved into the biological sample as expected, so as to be able to sufficiently bind to the protein of interest in the biological sample. The detection reagent may also take other solid forms. For example, the fluid receiving chamber 104 may have a matrix material therein, such as a sponge or similar structure, and the detection reagent may be dispersedly attached in the matrix material (different from the separation matrix material described below). When the biological sample is added into the fluid receiving chamber 104, the detection reagent attached to the matrix material can be quickly dissolved into the biological sample. Alternatively, the detection reagent can also take a solution form, and when the fluid receiving chamber 104 receives the biological sample, the solution of the detection reagent can also be quickly and sufficiently mixed with the biological sample.

When passing through the fluid receiving chamber 104, the detection reagent is sufficiently mixed with the biological sample to increase the degree of binding of the detection reagent to the protein of interest in the biological sample. In some embodiments, the fluid receiving chamber may have a member to slow down the flow rate of the liquid, such as an hourglass or a serpentine fluid channel, to increase the time for the biological sample to flow through the fluid receiving chamber 104.

The fluid receiving chamber 104 can be in fluid communication with the downstream fluid separation chamber 106 through a fluid channel 114, so as to allow the biological sample mixed with the detection reagent to flow into the fluid separation chamber 106. In some embodiments, the fluid channel 114 can be sealed by a hydrolyzable membrane (the detection reagent is in a solid form), which can retain the detection reagent in a solid form in the fluid receiving chamber 104, thereby preventing the detection reagent from undesirably entering the fluid separation chamber 106 before detection begins. In other embodiments, the fluid channel 114 can also be sealed by a sealing plug or a similar fluid-tight structure (the detection reagent may be in a solid or liquid form).

It can be understood that the fluid receiving chamber 104 is mainly used to receive and introduce the biological sample, and make the biological sample sufficiently mixed with the detection reagent. In some embodiments, the housing 102 may also not have the fluid receiving chamber 104, in which case the biological sample may be pre-mixed with the detection reagents outside the housing 102. In this way, the mixed biological sample may be injected directly into the housing 102 via the fluid inlet 110, for example flowing directly into the fluid separation chamber 106. Wherein for the mixed biological sample, if the protein of interest exists therein, then the protein of interest sufficiently binds to the detection reagent, and the amount of the free form detection reagent in the biological sample is significantly reduced.

Still referring to Fig. 1, the fluid separation chamber 106 located downstream of the fluid receiving chamber 104 holds the separation matrix material described herein. When the biological sample flows through the fluid separation chamber 106, the separation matrix material can absorb the detection reagent in free state, preventing the detection reagent in free state from continuing to flow downstream, but does not affect other components flowing through the separation matrix material to continue to flow downstream. It can be understood that, in some embodiments, in order to increase the residence time of the biological sample in the fluid separation chamber 106, the separation matrix material can be held relatively tightly in the fluid separation chamber 106 to reduce the flow rate. In other embodiments, the fluid separation chamber 106 may also be constructed to have a member for slowing down the flow rate, such as an hourglass. In some embodiments, the fluid separation chamber 106 may have a fluid path of a predetermined length (for example, 1 cm, 2 cm, 5 cm, 10 cm or longer) to ensure sufficient time to process the biological sample.

As previously mentioned, the separation matrix material is capable of competing with the protein of interest or some other specific proteins for binding to the detection reagent, but the detection reagent has a lower affinity for the separation matrix material than for the protein of interest. That is to say, the detection reagent bound to the protein of interest will continue to flow downstream along with the biological sample, while other detection reagents in free state will be fixed in the fluid separation chamber 106 by the separation matrix material and will not move downstream.

Still referring to Fig. 1, after flowing through the fluid separation chamber 106, the biological sample continues to flow downstream. Specifically, the biological sample flows into the fluid collection chamber 108 through the fluid outlet 116 between the fluid separation chamber 106 and the fluid collection chamber 108. In some embodiments, the fluid outlet 116 may be provided with a removable sealing plug.

The fluid collection chamber 108 is used to collect the biological sample flowing through the fluid separation chamber 106. When there is a protein of interest bound to the detection reagent in the biological sample, the biological sample collected in the fluid collection chamber 108 will develop color due to the presence of the detection reagent. At least a portion of the fluid collection chamber 108 is light transmissive to allow the colored biological sample collected therein to be visually observed or optically detected. In some cases, the amount of biological sample may be small, so the height of the liquid collected in the fluid collection chamber 108 is limited, which affects the observation effect. Correspondingly, the fluid collection chamber can be constructed to have an internal cross-section (for example, a tapered cross-section) that tapers downward in a generally vertical direction, so as to increase the height of the liquid in the chamber for easy observation.

As can be seen from the embodiment shown in Fig. 1, the housing 102 can be placed such that the fluid receiving chamber 104, the fluid separation chamber 106 and the fluid collection chamber 108 are arranged from high to low when detection is performed. In this way, the gravity of the biological sample liquid itself can drive it to flow downstream, and flow through these chambers respectively to collect in the fluid collection chamber 108. It can be understood that in some other embodiments, the apparatus 100 may also have other fluid driving mechanisms to drive the liquid to flow in the housing 102 in sequence. For example, a removable fluid pump, piston or similar mechanism may be provided at the upper end of the most upstream fluid receiving chamber 104 of the housing 102. After the biological sample is added to the housing 102, the fluid pump or similar mechanism may be attached to the housing and pressurize the interior of the housing 102 such that the biological sample flows to the fluid separation chamber 106 and the fluid collection chamber 108.

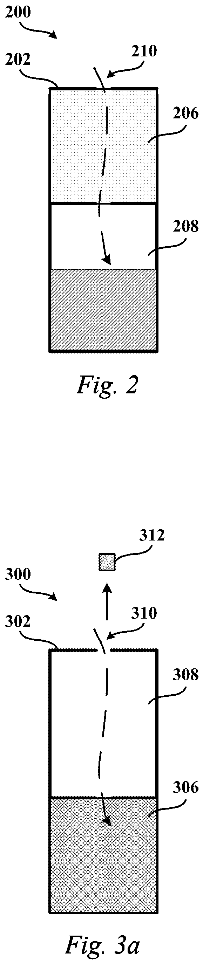

Fig. 2 shows an apparatus 200 for detecting a misfolded protein in a biological sample according to another embodiment of the present application. In contrast to the apparatus 100 shown in Fig. 1, the apparatus 200 shown in Fig. 2 does not comprise a fluid receiving chamber in the housing. Accordingly, the detection reagent can be provided separately, and the detection reagent and the biological sample are pre-mixed outside the housing. In this way, when the mixed biological sample is injected into the apparatus 200 through the fluid inlet 210 of the housing 202, it can directly enter the fluid separation chamber 206, and further be processed by the fluid separation chamber 206 and then collected in the fluid collection chamber 208.

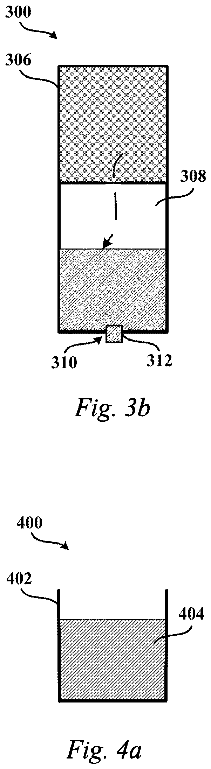

According to different usage methods, the detection apparatus of the present application can also adopt other suitable structures. Fig. 3a and Fig. 3b show an apparatus 300 for detecting a misfolded protein in a biological sample according to another embodiment of the present application, and Fig. 3a and Fig. 3b are schematic diagrams of the detection apparatus 300 in different states or stages, respectively.

As shown in Fig. 3a, the apparatus 300 comprises a housing 302, wherein the housing 302 further comprises a fluid separation chamber 306, and a fluid collection chamber 308 in fluid communication with the fluid separation chamber 306. Depending on the different stages of the detection process, the fluid collection chamber 308 is also used as a fluid receiving chamber, which will be described in detail below.

Specifically, the fluid collection chamber 308 may be preloaded with the detection reagent. Referring to Fig. 3a, the fluid collection chamber 308 is placed above the fluid separation chamber 306 at the beginning of the detection. Similar to the fluid receiving chamber 104 shown in Fig. 1, the biological sample is injected into the fluid collection chamber 308 through the fluid inlet 310 (at this time, the fluid collection chamber 308 is used as a fluid receiving chamber), and is sufficiently mixed with the detection reagent loaded in the fluid collection chamber 308. Afterwards, under the action of gravity, the mixed biological sample flows from the fluid collection chamber 308 into the fluid separation chamber 306 through the fluid channel, and takes substantially all of the detection reagent in the fluid collection chamber 308 with it. In the fluid separation chamber 306, the separation matrix material therein binds and adsorbs the free detection reagent in the biological sample, thereby retaining the free detection reagent therein.

Next, as shown in Fig. 3b, after placing the apparatus 300 in the direction shown in Fig. 3a for a short period of time (for example, 5 seconds, 10 seconds, 20 seconds, 30 seconds, 2 minutes or more), the fluid inlet 310 is sealed with a sealing plug 312 to prevent liquid from flowing out of the housing 302. Next, the apparatus 300 is inverted such that the fluid collection chamber 308 is placed below the fluid separation chamber 306. In this way, under the action of gravity, the biological sample will flow from the fluid separation chamber 306 back to the fluid collection chamber 308 via the fluid channel, and be collected therein. It can be understood that since substantially all of the pre-existing detection reagent in the fluid collection chamber 308 has been dissolved in the biological sample and brought into the fluid separation chamber 306, no detection reagent remains in the fluid collection chamber 308. In some embodiments, the amount of the detection reagent contained in fluid collection chamber 308 does not exceed the amount of the detection reagent that can be retained (i.e., bound) in the fluid separation chamber 306.

In some embodiments, the detection reagent may not be preloaded in the fluid collection chamber 308, but pre-mixed with the biological sample outside the apparatus 300, or added to the fluid collection chamber 308 separately or simultaneously with the biological sample.

It can be seen that, compared with the apparatus 100 shown in Fig. 1, the apparatus 300 shown in Fig. 3a and 3b has a simpler structure and lower manufacturing and use costs.

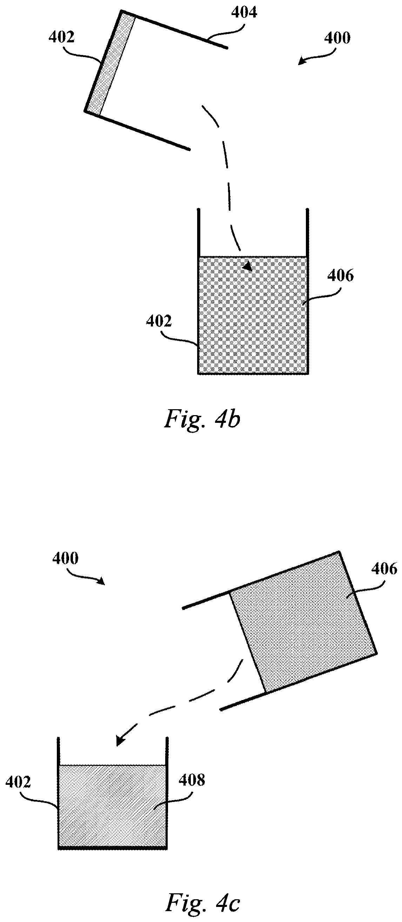

Fig. 4a, Fig. 4b and Fig. 4c show an apparatus 400 for detecting a misfolded protein in a biological sample according to yet another embodiment of the present application, and they are schematic diagrams of the detection apparatus 400 in different states or stages, respectively.

As shown in Fig. 4a to Fig. 4c, compared with the one-piece structure shown in Fig. 1 to Fig. 3b, the housing 402 of the apparatus 400 adopts a split structure that comprises a plurality of chambers that are independently constructed and separated from each other. However, each chamber has an opening to allow liquid to flow in and/or out. Next, the apparatus 400 will be further described in connection with the sequence of actual detection or use.

As shown in Fig. 4a, the housing 402 comprises a fluid receiving chamber 404. In some embodiments, the fluid receiving chamber 404 is used to receive the biological sample, and mix the detection reagent added in advance or during detection with the biological sample, so that the protein of interest in the biological sample can sufficiently bind to the detection reagent.

Furthermore, as shown in Fig. 4b, the housing 402 also comprises a fluid separation chamber 406 containing the separation matrix material therein. A user may invert the fluid receiving chamber 404 to pour the mixed biological sample from the fluid receiving chamber 404 into the fluid separation chamber 406. In this way, the detection reagent in the free state can be separated from the biological sample by the process of the separation matrix material, and adsorbed and bound in the separation matrix material.

As shown in Fig. 4c, the housing 402 further comprises a fluid collection chamber 408 for collecting the biological sample processed by the separation matrix material. A user may invert the fluid separation chamber 406 to pour the mixed biological sample from the fluid separation chamber 406 into the fluid collection chamber 408. If the original biological sample contains the protein of interest, it binds to the detection reagent during the detection process, and finally carries the detection reagent and is collected in the fluid collection chamber 408 and develops color therein. It can be understood that in some embodiments, the fluid collection chamber 408 can share a chamber with the fluid receiving chamber, that is, one chamber functions as a fluid receiving chamber at the beginning of the detection and functions as a fluid collection chamber at the later stage of the detection. In other embodiments, no fluid receiving chamber may be provided, but the detection reagent and the biological sample are pre-mixed outside the apparatus 400.

The apparatus for detecting a misfolded protein in a biological sample according to the above-described embodiments of the present application can be provided as a kit.

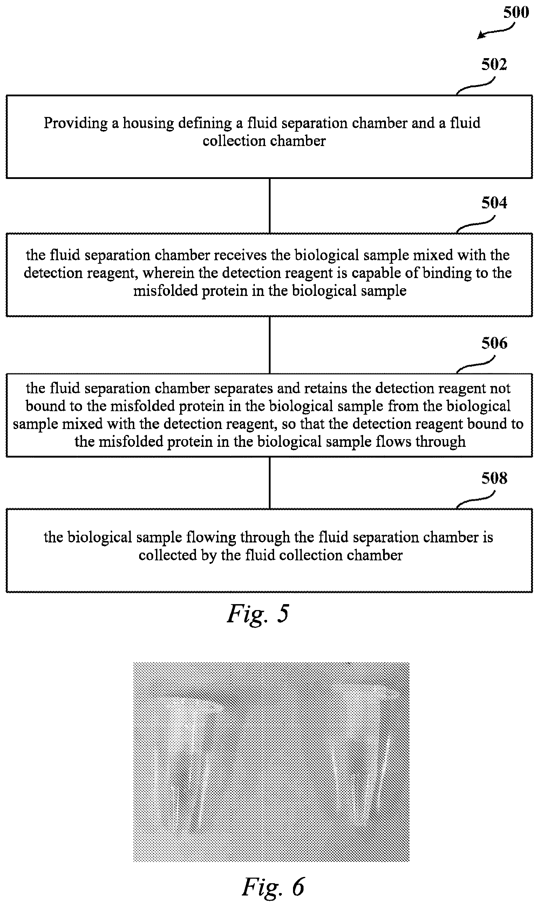

Fig. 5 shows a method 500 for detecting a misfolded protein in a biological sample according to one embodiment of the present application. As shown in Fig. 5, the method 500 comprises: In step 502, a housing is provided that defines a fluid separation chamber and a fluid collection chamber; In step 504, the fluid separation chamber receives the biological sample mixed with the detection reagent, wherein the detection reagent is capable of binding to the misfolded protein in the biological sample; In step 506, the fluid separation chamber separates and retains the detection reagent not bound to the misfolded protein in the biological sample from the biological sample mixed with the detection reagent, so that the detection reagent bound to the misfolded protein in the biological sample flows through; and in step 508, the biological sample flowing through the fluid separation chamber is collected by the fluid collection chamber.

It can be understood that the apparatuses 100-400 in the above embodiments of the present application can be used to implement the method for detecting the misfolded protein in the biological sample.

In one aspect, a method for detecting a misfolded protein or an aggregate thereof in a biological sample is provided and the method comprises: providing a housing defining a fluid separation chamber and a fluid collection chamber; the fluid separation chamber receives the biological sample mixed with the detection reagent, wherein the detection reagent is capable of binding to the misfolded protein or the aggregate thereof; the fluid separation chamber separates and retains the detection reagent not bound to the misfolded protein or the aggregate thereof from the biological sample mixed with the detection reagent, so that the detection reagent bound to the misfolded protein or the aggregate thereof flows through; and the biological sample flowing through the fluid separation chamber is collected by the fluid collection chamber.

In some embodiments, the housing further defines a fluid receiving chamber, and the fluid separation chamber is located between the fluid receiving chamber and the fluid collection chamber. Further, the method further comprises: before the biological sample mixed with the detection reagent is received by the fluid separation chamber, the biological sample mixed with the detection reagent is received by the fluid receiving chamber, and the biological sample flows into the fluid separation chamber via the fluid receiving chamber.

In some embodiments, the housing further defines a fluid receiving chamber, and the fluid separation chamber is located between the fluid receiving chamber and the fluid collection chamber, and the fluid receiving chamber contains the detection reagent. Further, the method further comprises: before the fluid separation chamber receives the biological sample mixed with the detection reagent, the biological sample is received by the fluid receiving chamber and mixed with the detection reagent, so that the biological sample mixed with the detection reagent flows into the fluid separation chamber via the fluid receiving chamber.

In some embodiments, the method further comprises: before the biological sample mixed with the detection reagent is received by the fluid separation chamber, the biological sample mixed with the detection reagent is received by the fluid collection chamber, and the biological sample flows into the fluid separation chamber via the fluid collection chamber.

In some embodiments, the fluid collection chamber contains the detection reagent. the method further comprises: before the fluid separation chamber receives the biological sample mixed with the detection reagent, the biological sample is received by the fluid collection chamber and mixed with the detection reagent, so that the biological sample mixed with the detection reagent flows into the fluid separation chamber via the fluid collection chamber.

In some embodiments, the fluid receiving chamber is in fluid communication with the fluid separation chamber.

In some embodiments, the collected biological sample can freely flow in the fluid collection chamber. In some further embodiments, the volume of freely flowing liquid in the fluid collection chamber is at least about 50 µL, at least about 100 µL, at least about 500 µL, at least about 1 mL, at least about 3 mL, or about 3 mL to about 5 mL.

In one aspect, the present disclosure also provides a method for predicting the risk of a subject suffering from a disease characterized by a misfolded protein, the method comprises detecting the presence of a misfolded protein in a biological sample according to the method of the present disclosure or using the apparatus of the present disclosure, wherein the presence of the misfolded protein indicates that the subject has or is at risk of having the disease characterized by the misfolded protein.

In addition, the apparatus and method of the present disclosure may also be used in a method of diagnosing preeclampsia or performing differential diagnosis in a patient suffering from gestational hypertension or a patient who may be suspected of having preeclampsia, and the method comprises the steps of: a) obtaining a biological sample (such as urine) from the patient with gestational hypertension, and b) detecting the presence of a misfolded protein in the biological sample using the method or apparatus of the present disclosure, thereby providing or supporting the diagnosis of preeclampsia or acting as a differential diagnosis in the patient. In some embodiments, the present disclosure also comprises a method of treating a pregnant mammal suspected of having preeclampsia, and the method comprises: a) detecting the presence of a misfolded protein in a biological sample from the mammal using the method or apparatus of the present disclosure, thereby determining the presence of the misfolded protein indicating that the pregnant mammal has preeclampsia; and b) the pregnant mammal give birth to treat preeclampsia.

### Embodiments

This disclosure contemplates comprising, but not limited to, the following embodiments, and all combinations of features described in the following embodiments.

Embodiment 1. A method for detecting a misfolded protein or an aggregate thereof in a biological sample, wherein the method comprises:
(a) providing a biological sample;
(b) mixing the biological sample with a detection reagent, the detection reagent being capable of binding to the misfolded protein or the aggregate thereof;
(c) enabling the biological sample mixed with the detection reagent to come into contact with and pass through a separation matrix, the separation matrix being constructed to adsorb the detection reagent that does not bind to the misfolded protein or the aggregate thereof and to allow passage of the detection reagent binding to the misfolded protein or the aggregate thereof;
(d) collecting, in a free liquid state, the biological sample passing through the separation matrix; and
(e) detecting the presence of the detection reagent in the collected biological sample in the free liquid state, wherein the presence of the detection reagent indicates the presence of the misfolded protein or the aggregate thereof in the biological sample.

Embodiment 2. The method according to embodiment 1, wherein the misfolded protein or the aggregate thereof is selected from a misfolded protein, a protein aggregate, a supramolecular protein aggregate, a misfolded protein fragment, a protein aggregate fragment, a supramolecular protein aggregate fragment and any combination thereof.

Embodiment 3. The method according to embodiment 1 or 2, wherein the misfolded protein or the aggregate thereof contains a beta sheet structure.

Embodiment 4. The method of any one of embodiments 1-3, wherein in step (b), the biological sample mixed with the detection reagent has a volume of at least about 0.5 mL, at least about 1 mL, at least about 3 mL, or about 3 mL to about 5 mL.

Embodiment 5. The method according to embodiment 4, wherein in step (d), the biological sample is collected in a fluid collection chamber, and the collected biological sample is in a free liquid state in the fluid collection chamber.

Embodiment 6. The method according to embodiment 5, wherein the collected biological sample can freely flow in the fluid collection chamber.

Embodiment 7. The method according to embodiment 6, wherein the volume of freely flowing liquid in the fluid collection chamber is at least about 50 µL, at least about 100 µL, at least about 500 µL, at least about 1 mL, at least about 3 mL, or about 3 mL to about 5 mL.

Embodiment 8. The method of any one of embodiments 5-7, wherein the fluid collection chamber does not provide capillary action.

Embodiment 9. The method of any one of embodiments 5-8, wherein the collected biological sample in the free liquid state is not attached or adsorbed or bound to the inner wall of the fluid collection chamber or substantially is not attached or adsorbed or bound to the inner wall of the fluid collection chamber.

Embodiment 10. The method of any one of embodiments 1-9, wherein the collected biological sample in the free liquid state allows the detection of step (e) to be performed by an optical method.

Embodiment 11. The method of any one of embodiments 1-10, wherein the detection of step (e) is performed by a visual method or an optical method.

Embodiment 12. The method of any one of embodiments 1-11, wherein the detection reagent is capable of specifically binding to the misfolded protein.

Embodiment 13. The method of any one of embodiments 1-12, wherein the detection reagent is a dye, such as a visible light dye or a fluorescent dye, such as an azo dye or an analog thereof (such as Congo red or Evans blue), a benzothiazole dye or an analog thereof (such as Thioflavin T and Thioflavin S), Amaranth red, Brilliant black, or Nile red.

Embodiment 14. The method of any one of embodiments 1-13, wherein the separation matrix comprises one or more materials selected from cotton or cotton gauze; silk; a cellulose, such as nitrocellulose, microcrystalline cellulose, cellulose acetate, or wood chips; a polymer, such as polyester, polyethylene, polysulfone, polyvinyl alcohol, polyethylene glycol (such as PEG2000, PEG3000, PEG4000, PEG5000 or PEG6000), or polyacrylamide; glass fiber; silica gel, gelatin or dextran gel; a dry protein or a protein dry powder, such as egg white protein dry powder; an inorganic mineral soil, such as zeolite, clay, kaolin, hydroxyapatite and montmorillonite; a calcium salt, such as calcium chloride, calcium carbonate or calcium phosphate; activated carbon; or activated alumina.

Embodiment 15. The method of any one of embodiments 1-14, wherein the separation matrix comprises activated alumina and the dye is Congo red or an analog thereof.

Embodiment 16. The method of any one of embodiments 1-15, wherein the biological sample is from a mammal, such as human, suspected of having or at risk of having a protein misfolding disorder.

Embodiment 17. The method according to embodiment 16, wherein the protein misfolding disorder is selected from the group comprising: preeclampsia, severe preeclampsia, atypical preeclampsia, eclampsia, Alzheimer's disease, cerebral beta-amyloid angiopathy, retinal ganglion cell degeneration in glaucoma, prion disease, Parkinson's disease and other synucleinopathy, tauopathies, frontotemporal lobar degeneration FTLD, FLTD_FUS, amyotrophic lateralizing sclerosis, Huntington's disease and other trilogy, repetition disorder, dementia (UK and Danish familial), hereditary cerebral hemorrhage with amyloidosis, CADASIL, Alexander disease, various amyloidosis, Serinopathies, Type II diabetes, inclusion body myositis/myopathy, cataract, Retinitis pigmentosa with rhodopsin mutation, medullary thyroid carcinoma, pituitary prolactinoma, hereditary lens corneal dystrophy, Mallory's bodies, pulmonary alveolar proteinosis, odontogenic neoplastic amyloidosis, cystic fibrosis, sickle cell disease and critical illness myopathy.

Embodiment 18. An apparatus for detecting a misfolded protein or an aggregate thereof in a biological sample, wherein the apparatus comprises:
a housing defining a fluid separation chamber and a fluid collection chamber,
wherein, the fluid separation chamber is used to receive a mixed liquid of the biological sample and a detection reagent, wherein the detection reagent is capable of binding to the misfolded protein or the aggregate thereof in the biological sample, the fluid separation chamber contains a separation matrix constructed to adsorb the detection reagent that does not bind to the misfolded protein or the aggregate thereof from the mixed liquid and to allow passage of the detection reagent binding to the misfolded protein or the aggregate thereof; and
the fluid collection chamber is used to collect the biological sample flowing through the separation matrix in the fluid separation chamber.

Embodiment 19. The apparatus according to embodiment 18, wherein the misfolded protein or the aggregate thereof is selected from a misfolded protein, a protein aggregate, a supramolecular protein aggregate, a misfolded protein fragment, a protein aggregate fragment, a supramolecular protein aggregate fragment and any combination thereof.

Embodiment 20. The apparatus according to embodiment 18 or 19, wherein the misfolded protein or the aggregate thereof contains a beta sheet structure.

Embodiment 21. The apparatus of any one of embodiments 18-20, wherein the detection reagent is capable of specifically binding to the misfolded protein.

Embodiment 22. The apparatus of any one of embodiments 18-21, wherein the detection reagent is a dye, such as a visible light dye or a fluorescent dye, such as an azo dye or an analog thereof (such as Congo red or Evans blue), a benzothiazole dye or an analog thereof (such as Thioflavin T and Thioflavin S), Amaranth red, Brilliant black, or Nile red.

Embodiment 23. The apparatus of any one of embodiments 18-22, wherein the separation matrix comprises one or more materials selected from : cotton or cotton gauze; silk; a cellulose, such as nitrocellulose, microcrystalline cellulose, cellulose acetate, or wood chips; a polymer, such as polyester, polyethylene, polysulfone, polyvinyl alcohol, polyethylene glycol (such as PEG2000, PEG3000, PEG4000, PEG5000 or PEG6000), or polyacrylamide; glass fiber; silica gel, gelatin or dextran gel; a dry protein or a protein dry powder, such as egg white protein dry powder; an inorganic mineral soil, such as zeolite, clay, kaolin, hydroxyapatite and montmorillonite; a calcium salt, such as calcium chloride, calcium carbonate or calcium phosphate; activated carbon; or activated alumina.

Embodiment 24. The apparatus of any one of embodiments 18-23, wherein the fluid collection chamber is constructed to collect, in a free liquid state, the biological sample flowing through the fluid separation chamber.

Embodiment 25. The apparatus according to embodiment 24, wherein the collected biological sample can freely flow in the fluid collection chamber.

Embodiment 26. The apparatus according to embodiment 25, wherein the volume of freely flowing liquid in the fluid collection chamber is at least about 50 µL, at least about 100 µL, at least about 500 µL, at least about 1 mL, at least about 3 mL, or about 3 mL to about 5 mL.

Embodiment 27. The apparatus of any one of embodiments 18-26, wherein the fluid collection chamber does not provide capillary action.

Embodiment 28. The apparatus of any one of embodiments 18-27, wherein the collected biological sample is not attached or adsorbed or bound to the inner wall of the fluid collection chamber or substantially is not attached or adsorbed or bound to the inner wall of the fluid collection chamber.

Embodiment 29. The apparatus of any one of embodiments 18-28, wherein at least a portion of the fluid collection chamber is light transmissive, so that the biological sample collected therein can be detected by an optical method.

Embodiment 30. The apparatus of any one of embodiments 18-29, wherein the apparatus further comprises an isolation layer disposed between the fluid separation chamber and the fluid collection chamber and separating the fluid separation chamber and the fluid collection chamber.

Embodiment 31. The apparatus according to embodiment 30, wherein the isolation layer allows the biological sample to flow through and prevents the separation matrix from entering the fluid collection chamber.

Embodiment 32. The apparatus according to embodiment 31, wherein the material of the isolation layer is selected from inorganic materials (such as inorganic fibers or particulates), such as ceramics, glass, glass fiber, or metals (such as stainless steel); a polymer, comprising a polymer fiber or a polymer particulate, such as a polyamide (such as nylon), a polyethylene (such as ultra-high molecular weight polyethylene (UHMW-PE), polytetrafluoroethylene (PTFE), polystyrene, or polyvinyl chloride (PVC)), a polyacrylic (such as acrylic), a polypropylene, a plastic (such as a porous plastic), a polyester and a polyurethane; a cellulose, such as filter paper or wood pulp cellulose, and optionally, the material is hydrophilic or has been subjected to a hydrophilic treatment.

Embodiment 33. The apparatus of any one of embodiments 30-32, wherein the isolation layer has a thickness of 0.1-5 mm and/or a pore size of 5-200 µm.

Embodiment 34. The apparatus of any one of embodiments 18-33, wherein the biological sample is from a mammal, such as human, suspected of having or at risk of having a protein misfolding disorder, for example, the biological sample is a sample selected from one or more of urine, blood, saliva, tissue fluid, interstitial fluid, serum, plasma, cerebrospinal fluid, or amniotic fluid.

Embodiment 35. The apparatus of any one of embodiments 18-34, wherein the housing further defines a fluid receiving chamber for receiving the biological sample.

Embodiment 36. The apparatus according to embodiment 35, wherein the fluid receiving chamber contains the detection reagent, and the biological sample is mixed with the detection reagent.

Embodiment 37. The apparatus according to embodiment 35 or 36, wherein the fluid separation chamber and the fluid receiving chamber are in fluid communication or separated.

Embodiment 38. The apparatus of any one of embodiments 35-37, wherein the fluid receiving chamber further comprises a member for slowing the flow rate of the fluid, such as an hourglass.

Embodiment 39. The apparatus of any one of embodiments 35-38, wherein a removable sealing plug is installed at the fluid inlet of the fluid receiving chamber.

Embodiment 40. The apparatus of any one of embodiments 18-34, wherein the detection reagent is contained in the fluid collection chamber, and the fluid collection chamber further comprises:
a sample inlet through which the fluid collection chamber receives the biological sample and allows the biological sample to mix with the detection reagent contained therein;
a fluid channel located between the fluid collection chamber and the fluid separation chamber to allow the biological sample mixed with the detection reagent to flow from the fluid collection chamber into the fluid separation chamber, and to allow the biological sample flowing through the fluid separation chamber to flow back into the fluid collection chamber; and
a sealing plug for removably sealing the sample inlet;
wherein the amount of the detection reagent contained in fluid collection chamber does not exceed the amount of the detection reagent that can be retained in the fluid separation chamber.

Embodiment 41. The apparatus of any one of embodiments 18-34, wherein the fluid collection chamber further comprises:
a sample inlet through which the fluid collection chamber receives the biological sample mixed with the detection reagent;
a fluid channel located between the fluid collection chamber and the fluid separation chamber to allow the biological sample mixed with the detection reagent to flow from the fluid collection chamber into the fluid separation chamber, and to allow the biological sample flowing through the fluid separation chamber to flow back into the fluid collection chamber; and
a sealing plug for removably sealing the sample inlet.

Embodiment 42. The apparatus of any one of embodiments 18-41, wherein the fluid collection chamber is constructed to have an internal cross-section that tapers downward in a generally vertical direction.

Embodiment 43. Use of the apparatus according to any one of embodiments 18-42 in the manufacture of a kit for diagnosing or predicting a disease characterized by a misfolded protein.

Embodiment 44. Use according to embodiment 43, wherein the disease is selected from the group comprising: preeclampsia, severe preeclampsia, atypical preeclampsia, eclampsia, Alzheimer's disease, cerebral beta-amyloid angiopathy, retinal ganglion cell degeneration in glaucoma, prion disease, Parkinson's disease and other synucleinopathy, tauopathies, frontotemporal lobar degeneration FTLD, FLTD_FUS, amyotrophic lateralizing sclerosis, Huntington's disease and other trilogy, repetition disorder, dementia (UK and Danish familial), hereditary cerebral hemorrhage with amyloidosis, CADASIL, Alexander disease, various amyloidosis, Serinopathies, Type II diabetes, inclusion body myositis/myopathy, cataract, Retinitis pigmentosa with rhodopsin mutation, medullary thyroid carcinoma, pituitary prolactinoma, hereditary lens corneal dystrophy, Mallory's bodies, pulmonary alveolar proteinosis, odontogenic neoplastic amyloidosis, cystic fibrosis, sickle cell disease and critical illness myopathy.

Embodiment 45. The use according to embodiment 44, wherein the disease is preeclampsia.

Embodiment 46. The use according to embodiment 44, wherein the disease is Alzheimer's disease or Parkinson's disease.

Embodiment 47. A method of predicting the risk of suffering from a disease characterized by a misfolded protein, wherein the method comprises detecting the presence of a misfolded protein in a biological sample by the method according to any one of embodiments 1-17 or using the apparatus according to any one of embodiments 18-42.

Embodiment 48. Use of a combination of (1) a dye capable of specifically binding to a misfolded protein and (2) a separation column loaded with an adsorbent material capable of adsorbing the dye in a free state in preparing a kit for diagnosing or predicting a disease characterized by the misfolded protein.

Embodiment 49. The use according to embodiment 48, wherein the separation column is located in a fluid separation chamber defined by a housing, and the housing further defines a fluid collection chamber in fluid communication with the fluid separation chamber.

It should be noted that although several steps or modules of the apparatus and method for detecting whether there is a misfolded protein in a biological sample are mentioned in the above detailed description, this division is only exemplary and not mandatory. Actually, according to the embodiments of the present application, the features and functions of two or more modules described above may be embodied in one module. Conversely, the features and functions of one module described above can be further divided into and embodied by a plurality of modules.

### Examples

### Example 1 Dye test

Different types of dyes were tested, comprising Thioflavin T, Thioflavin S, Congo red, Evans blue, Amaranth red, Brilliant black and Nile red. The test results use "excellent", "good", "medium" and "poor" to indicate the applicability of the dyes. "Excellent" indicates that the dye can bind to the misfolded protein in the sample, can be separated from a free dye, and has a color easy to observe; and the test repeatability is good. "Good" indicates that the dye can bind to the misfolded protein in the sample, can be separated from a free dye, and has a color easy to observe; and the test repeatability is moderate. "Medium" indicates that the dye can bind to the misfolded protein in the sample, can be separated from a free dye, and has a color not easy to observe; and the test repeatability is moderate. "Poor" means unavailable. The test results are shown in the table below.

| **Dye** | **Manufacturer** | **Batch No.** | **Result** |
|---|---|---|---|
| Thioflavin S | Sigma | SLBG6233V | Good |
| Thioflavin T | Sigma | T1892 | Good |
| Congo red | Sigma | MKBX816J | Excellent |
| Evans blue | ALADDIN | A1309044 | Good |
| Amaranth red | Sigma | MKCF8543 | Good |
| Brilliant black | Tokyo Chemical Industry Co., Ltd. | 4QWMK-RB | Good |
| Nile red | Shanghai Yuanye Bio-Technology Co., Ltd | SJ06246AB | Good |

### Example 2 Separation material test

Various separation materials were tested. The test results use "excellent", "good", "medium" and "poor" to indicate the applicability of the material. "Excellent" indicates that the material can distinguish free dyes from bound dyes; the flow rate of the sample in the material is appropriate; filling of the material is convenient; the material can filter out the background color of urine; and the test repeatability is good. "Good" indicates that the material can distinguish free dyes from bound dyes; the flow rate of the sample in the material is appropriate; the test repeatability is good; the filling convenience of the material is moderate; the material cannot filter out the color of urine. "Medium" indicates that the material can distinguish free dyes from bound dyes; the flow rate of the sample in the material is appropriate; the test repeatability is moderate; the filling convenience of the material is moderate; the material cannot filter out the color of urine. "Poor" means unavailable.

| **Manufacturer** | **Model/Specification** | **Materials** | **Result** | **Dosage** |
|---|---|---|---|---|
| Fuda Sanitary Material Factory | 300 g | Cotton | Good | 0.1 g |
| Fuda Sanitary Material Factory | 500 g | Cotton gauze | Good | 0.1 g |
| Sinopharm Chemical Reagent Co., Ltd. | 30153060-250 g | Polyvinyl alcohol | Good | 0.6 g |
| Sigma | 06300-1 kg | Acid alumina | Excellent | 0.8 g |
| Shanghai Xinfan Biology Science and Technology Co., Ltd GEMIC | 40-120 µm | Dextran gel | Good | 0.5 g |
| | 100 g | | | |
| Sinopharm Chemical Reagent Co., Ltd. | 10006619-500 g | Activated carbon | Good | 0.2 g |
| Sinopharm Chemical Reagent Co., Ltd. | 68005761-250 g | Microcrystalline cellulose | Excellent | 0.3 g |
| Sinopharm Chemical Reagent Co., Ltd. | 69007260-250 g | Cellulose acetate | Excellent | 0.3 g |
| Sinopharm Chemical Reagent Co., Ltd. | 20034361-500 g | Silica gel | Good | 0.6 g |
| Sinopharm Chemical Reagent Co., Ltd. | P8240-500 g | PEG4000 | Good | 0.8 g |
| Aishangta Pet Products Shop | 800 g | Sawdust | Good | 0.1 g |
| Shanghai Qiangshun Chemical Co., Ltd. | 40-60 mesh 250 g | Zeolite | Good | 0.3 g |
| Homemade | Homemade | Dry protein (duck egg white protein dry powder) | Excellent | 0.3g |
| Dimiao House | 500 g | Clay | Good | 1.0 g |
| Fuchen (Tianjin) Chemical Reagent Co., Ltd. | 500 g | Kaolin | Good | 0.9 g |
| Tianjin Zhiyuan Chemical Reagent Co., Ltd. | 500 g | Calcium chloride anhydrous | Good | 0.6 g |
| Shanghai Macklin Biochemical Co., Ltd | 80 µm | Hydroxyapatite | Good | 0.7 g |
| | H875582-25 g | | | |
| Sinopharm Chemical Reagent Co., Ltd. | MW 3 million 250 g | Polyacrylamide, anionic | Good | 0.5 g |
| Sigma | G7041-500 g | Gelatin | Good | 0.4 g |
| Shanghai Aladdin Biochemical Technology Co., Ltd. | M109698-50 g | Montmorillonite | Good | 0.7 g |
| Sinopharm Chemical Reagent Co., Ltd. | 10005717-250 g | Calcium carbonate | Good | 0.6 g |

### Example 3 Isolation material test

The following various isolation materials were tested. The test results use "excellent", "good", "medium" and "poor" to indicate the applicability of the material. "Excellent" means that the material can have better isolation and loading function; the flow rate of the sample in the material is appropriate; convenient to fill; and the test repeatability is good. "Good" means that the material can have better isolation and loading function; the flow rate of the sample in the material is appropriate; the test repeatability is good; the filling convenience of the material is moderate. "Medium" means that the material can have moderate isolation and loading function; the flow rate of the sample in the material is moderate; the filling convenience of the material is moderate. "Poor" means unavailable.

| **Name** | **Manufacturer** | **Item No.** | **Specification** | **Main materials** | **Effects** |
|---|---|---|---|---|---|
| Sieve plate | Hangzhou Cobetter Filtration Equipment Co., Ltd. | PM 100 H 0765 NIDR 015; PM 100 H 0700 NIDR 015 | 100 µm | PE particles | Excellent |
| Nylon mesh | Hangzhou Cobetter Filtration Equipment Co., Ltd. | 200 mesh | 200 mesh | Nylon 6 | Medium |
| Glass fiber filter paper | Shanghai Kinbio Tech Co., Ltd. | SB08 | 300 × 200 mm | Glass fiber | Excellent |
| Filter paper | Fushun Civil Administration Filter Paper Factory, Liaoning province | 11 cm round | Fast speed: 80-120 µm | Wood pulp cellulose | Good |
| | | | Medium speed: 30-50 µm | | |

### Example 4 Congo red dye test

In order to further test the performance of the dye Congo red, a 1 mg/mL human plasma albumin HSA solution was prepared and heated at 90°C for 30 minutes and used as a simulated positive sample. A 5 mg/mL Congo red solution was prepared as the dye stock solution. 0.2 g of alumina was used as the separation material to make a separation column, 1 mL of 1 mg/mL HSA solution was taken and used as a simulated positive sample, and 1 mL of water was taken and used as a simulated negative sample, and 5 µL of Congo red dye stock solution was added, respectively. The simulated negative sample and the simulated positive sample were passed through the alumina separation column respectively, and the color of the collected liquid was observed. The results showed that the collected liquid of the simulated positive sample was red, and the collected liquid of the simulated negative sample was colorless, as shown in Fig. 6.

### Example 5 Alumina filtration urine color test.

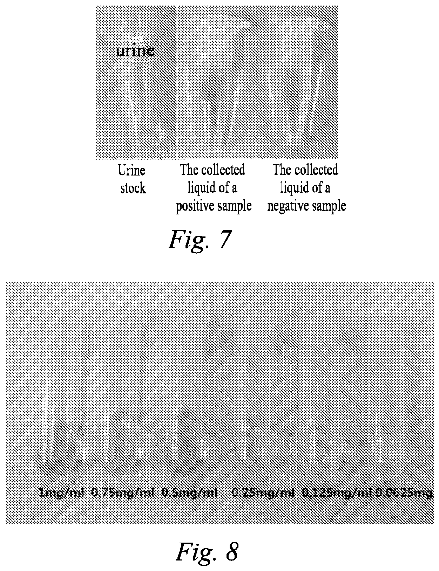

0.2 g of alumina was used as the separation material to make a separation column, 1 mL each of the collected negative urine sample and the collected positive urine sample was taken, and 5 µL of 5 mg/mL Congo red solution was added, respectively. The prepared samples were passed through the alumina separation column respectively, and the color of the collected liquid was observed. The results showed that the collected liquid of the positive sample was red, and the collected liquid of the negative sample was colorless and transparent, as shown in Fig. 7.

### Example 6 Sensitivity test of separation matrix material detection

0.2 g of alumina was used as the separation material to make a separation column. Human serum albumin HSA gradient solution (1 mg/mL, 0.75 mg/mL, 0.5 mg/mL, 0.25 mg/mL, 0.125 mg/mL, 0.0625 mg/mL) was prepared. After heating at 90°C for 30 minutes, 1 mL of each prepared HSA gradient solution was taken and 5 µL of 5 mg/mL Congo red solution was added, respectively. The resulting solutions were passed through the alumina separation column respectively, and the color of the collected liquid was observed. The results showed that when the concentration of HSA in the sample was 0.25 mg/mL, the naked eye can see obvious red color, and when the concentration was 0.125 mg/mL, the collected liquid is light red, that is, the detection sensitivity can reach at least 0.25 mg/mL or even lower. The results are shown in Fig. 8.

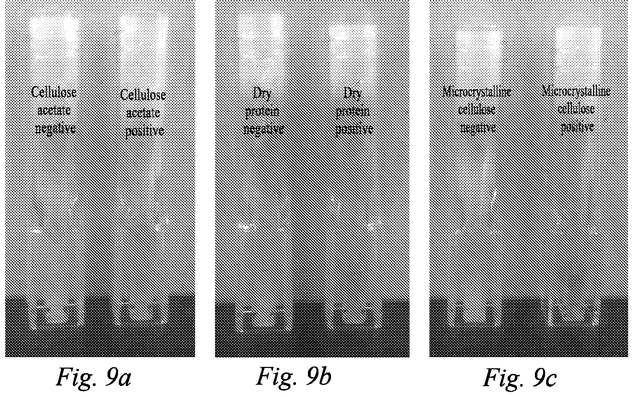

0.3 g of cellulose acetate, 0.3 g of dry protein (duck egg white protein dry powder), and 0.3 g of microcrystalline cellulose were used as separation materials to make separation columns, respectively. Human serum albumin HSA solution (10 mg/mL) was prepared. After heating at 90°C for 30 minutes, denatured HSA was taken and diluted to 0.3 mg/mL, totaling 1 ml, and 7 µL of 5 mg/mL Congo red solution was added. The resulting solution was passed through the separation column respectively, and the color of the collected liquid was observed. The results showed that when denatured HSA was contained, obvious red color can be observed by the naked eye, and when denatured HSA was not contained, the effluent was colorless. The results are shown in Fig. 9a-9c. The above results also indicated that the three separation media tested can also achieve detection sensitivities at least comparable to those of activated alumina.

### Example 7 Adsorption and separation of Congo red dye by alumina

Purpose: In the present invention, the separation of Congo red dye by the selected separation material alumina was based on an adsorption mechanism and this experiment was carried out to verify this mechanism.

Formulation materials: 4 g of anhydrous copper sulfate (Tianmao Chemical, lot: 20200315) was weighed, and diluted to 250 mL with deionized water, and the concentration of the obtained copper sulfate solution was 0.1 M. 5 mg of Congo red (Sigma, lot: MKBX8167V) was weighed, 1 mL of deionized water was added, shaken well to form a homogeneous solution, and the concentration of the prepared Congo red solution was 5 mg/mL. To prepare a simulated positive sample, 10 mg of human serum albumin (HSA) (Solarbio, lot: 7241052) was weighed, 1 mL of deionized water was added to prepare a 10 mg/mL HSA solution, which was heated in a boiling water bath at 90°C for 30 min, and then returned to room temperature for use. In order to prepare a separation apparatus, 0.8 g of alumina was weighed and added to a homemade separation column.

Detection: Copper sulfate solution (a solution without denatured proteins) was used as the simulated negative sample. The copper sulfate solution itself was blue, but after adding Congo red solution, the solution became nearly black. 1 mL of copper sulfate solution was taken, 7 µL of Congo red solution was added, shaken well and added to the separation column; after separation by the separation column, the color of the collected liquid was blue. The absorbance of the collected liquid was measured at 490 nm (characteristic absorption band of free Congo red) and 800 nm (characteristic absorption band of copper sulfate solution). The absorbance at 490 nm was 0.001, while the absorbance at 800 nm was 0.021, that is, there was no Congo red in the collected liquid. This showed that Congo red in the above mixed liquid was still retained in the alumina separation column, while copper sulfate was in the collected liquid.

A simulated positive sample (denatured HSA solution) was taken and diluted to 1 mg/mL, and then 1 mL of the diluted solution was taken, and 7 µL of Congo red solution was added and shaken well, and the resulting mixed liquid was red in color and added to the separation column. After separation by the separation column, the color of the collected liquid was light red, which indicated that part of Congo red was not adsorbed by alumina because of its binding to denatured proteins, while excessive free Congo red was adsorbed by alumina. The absorbance of the collected liquid was measured at 520 nm, and the absorbance was 0.038, indicating that the collected liquid contained Congo red dye in a bound state.

Conclusion: The alumina used in the present invention can distinguish the negative sample from the positive sample by separating free dyes from bound dyes by alumina. In this experiment, the blue color of the copper sulfate solution was formed by copper ions, and the relative mass of copper was much smaller than the dye Congo red. After the simulated negative sample was separated by the separation column, if the separation mechanism was the molecular sieve effect, then Congo red should flow out before copper ions. In this experiment, the color of the collected liquid was blue, and there was no characteristic absorption formed by Congo red when measured at 490 nm; In the simulated positive sample, the collected liquid was red. This is because the Congo red dye was bound to the denatured protein and brought into the solution with the denatured protein, thus making the solution appear red, and forming the characteristic light absorption of the bound Congo red dye at 520 nm. Based on this, the separation of free dyes by alumina was based on the adsorption of dyes, rather than the molecular sieve effect.

### Example 8. Clinical study

In this experiment, test reagents were used to detect the urine samples of the subjects, and the detection results were compared with the gold standard determination results and statistically analyzed to verify the clinical application value of the test reagents. The minimum number of subjects enrolled in each group should meet the requirements of statistical analysis.

### Clinical trial group

| **No.** | **Classification** | **Minimum study sample size** |
|---|---|---|
| 1 | Negative group | 700 |
| 2 | Positive group | 100 |

### 1 Comparison method

The diagnostic criteria for preeclampsia in the "Chinese Medical Association Guidelines for the Diagnosis and Treatment of Hypertensive Disorders in Pregnancy (2015)" were used as the gold standard.

### 2 Selection of study subjects

This study was applicable to pregnant women who are 18 years old and above and whose gestational age was ≥ 20 weeks. The subjects were grouped according to the gold standard diagnostic results.

### 2.1 Positive group

Subjects diagnosed with preeclampsia according to the "Chinese Medical Association Guidelines for the Diagnosis and Treatment of Hypertensive Disorders in Pregnancy (2015)" were enrolled;

### 2.2 Negative group

Subjects diagnosed with non-preeclampsia according to the "Chinese Medical Association Guidelines for the Diagnosis and Treatment of Hypertensive Disorders in Pregnancy (2015)" were enrolled.

### 3 Selection of clinical trial institutions

In accordance with the regulations such as "Provisions For In-Vitro Diagnostic Reagent Registration", "Technical Guidelines for Clinical Trials of In-Vitro Diagnostic Reagents" and other regulations and the requirements of clinical sample size, at the same time in combination with factors such as epidemiological background and according to the catalogue of national drug clinical trial institutions approved by National Medical Products Administration, two or more medical institutions with clinical trial qualifications were selected as clinical study units.

### 4 Selection of clinical samples

### 4.1 Sample type

Urine;

### 4.2 Sample collection, storage and transportation

1) According to the inclusion criteria, subjects who meet the requirements were selected.
2) Urine samples were collected in obstetrics.
3) 5 ml of midstream urine from the enrolled subject was taken and placed in a collection tube;
4) The storage and transportation conditions of the samples should meet the requirements of the instructions;
5) Refrigerated or frozen samples should be slowly brought to room temperature before detection.

### 4.3 Clinical record

The researchers collected samples from subjects who met the inclusion criteria, and recorded the basic information of the subjects, date of sample collection, operator, *etc.*

### 4.4 Sample inclusion criteria

1) Volunteer to participate in this trial and sign the informed consent form;
2) Age ≥ 18 years old;
3) Gestational week ≥ 20 weeks;

### 4.5 Sample exclusion criteria

1) Visible hematuria to the naked eye;
2) Patients who cannot complete the gold standard diagnosis of preeclampsia.

### 4.6 Sample rejection criteria

1) Samples with traceability information missed;
2) Samples that did not meet the sample collection requirements or that were not collected in sufficient quantities should be rejected;
3) Subjects who cannot be detected due to errors in collection, storage, and trial operations and had difficulty to re-collect samples should be rejected;
4) Sample contamination, corruption, degradation, *etc.* caused by any factors would increase the uncertainty of the detection results and should be rejected;
5) Duplicate samples of the same subject should be rejected;
6) Subjects who were considered by the researchers to be inappropriate to continue participating in this clinical study.

### 5 Implementation of clinical trials

### 5.1 Sample detection

### Sample preparation

About 3-5 mL of midstream urine was collected in a clean container for immediate use or stored at 2-8°C until use. The urine sample was brought to room temperature before use.

### Detection kit

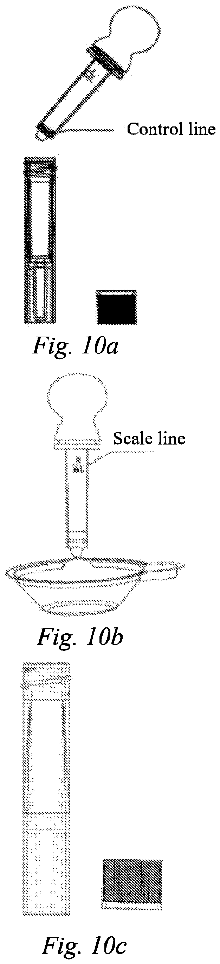

The structure of the detection kit was shown in Fig. 10a-10c, in which 0.8 g of acidic alumina was used as the separation matrix, Congo red was used as the detection reagent, and β sheet was used as the positive control.

### Test steps

(1) Before use, the kit and sample returned to room temperature.
(2) The cover of the test tube was unscrewed, and the test tube was placed upright on a flat surface (Fig. 10a).
(3) The rubber ball of the pipette was completely squeezed, the pipette was inserted into the urine collected in the container, and the urine was pipetted to the scale line (Fig. 10b). Note: the urine sample would turn red in the pipette.
(4) The rubber ball of the pipette was squeezed slowly, and the urine sample was loaded into the separation matrix in the test tube. The cover was tightly closed again, and the test tube was allowed to stand upright for about 3-10 minutes, then the color of the eluent in the test tube and the control line in the pipette was observed (Fig. 10c).

### Test results

(1) Negative result: the lower part in the test tube was a colorless effluent. The negative result indicated that the pregnant woman may not have preeclampsia at the time of the test.
(2) Positive result: the effluent at the lower part in the test tube was red. The positive result indicated that the pregnant woman may have preeclampsia and should be further consulted with a doctor.
(3) Invalid result: during a successful test, the control line in the pipette shown in Fig. 10c turned red. If the control line did not turn red, the test was repeated using another pipette and test tube. If the effluent was yellow-brown, the urine sample was not suitable for detection and the detection result was invalid.

### 5.2 Data summarization

After the clinical trial was completed, the clinical data was summarized, and the summary results were be submitted to the statisticians for statistical analysis.

### 6 Clinical evaluation method

The detection results of the test reagents were compared with the clear interpretation results of the gold standard, and the sensitivity, specificity, accuracy, negative predictive value, and positive predictive value of the reagents were observed.

| | | Clinical outcomes | | |
|---|---|---|---|---|
| | | Positive | Negative | |
| Detection results of the kit of the present invention | Positive | 107 | 13 | 120 |
| | Negative | 37 | 780 | 817 |
| | Total | 144 | 793 | 937 |

According to a study on a cohort of 937 subjects, the kit of the present invention achieved a sensitivity of 74.3%, a specificity of 98.4%, an accuracy of 94.7%, a positive predictive value (PPV) of 89.2%, and a negative predictive value (NPV) of 95.5%. Clinical data showed that the kit of the present invention achieved very high specificity and accuracy while taking sensitivity into account.

Those skilled in the art can understand and implement other changes to the disclosed embodiments by studying the specification, the disclosure, the drawings and the appended claims. In the claims, the word "comprise" does not exclude other elements and steps, and the words "a" and "an" do not exclude a plurality. In the actual use of the present application, one part may perform the functions of multiple technical features cited in the claims. Any reference signs in the claims should not be construed as limiting the scope. All publications, patents and patent applications mentioned in this specification are herein incorporated by reference to the same extent as if each individual publication or patent application is specifically and individually indicated to be incorporated by reference.

## Claims

1. A method for detecting a misfolded protein or an aggregate thereof in a biological sample, wherein the method comprises:
(a) providing a biological sample;
(b) mixing the biological sample with a detection reagent, the detection reagent being capable of binding to the misfolded protein or the aggregate thereof;
(c) enabling the biological sample mixed with the detection reagent to come into contact with and pass through a separation matrix, the separation matrix being constructed to adsorb the detection reagent that does not bind to the misfolded protein or the aggregate thereof and to allow passage of the detection reagent binding to the misfolded protein or the aggregate thereof;
(d) collecting, in a free liquid state, the biological sample passing through the separation matrix; and
(e) detecting the presence of the detection reagent in the collected biological sample in the free liquid state, wherein the presence of the detection reagent indicates the presence of the misfolded protein or the aggregate thereof in the biological sample.

2. The method according to claim 1, wherein in step (d), the biological sample is collected in a fluid collection chamber, and the collected biological sample is in a free liquid state in the fluid collection chamber.

3. The method according to claim 2, wherein the collected biological sample can freely flow in the fluid collection chamber.

4. The method according to claim 3, wherein the liquid that can freely flow in the fluid collection chamber has a volume of at least about 50 µL, at least about 100 µL, at least about 500 µL, at least about 1 mL, at least about 3 mL, or about 3 mL to about 5 mL.

5. The method according to any one of claims 2-4, wherein the fluid collection chamber does not provide capillary action.

6. The method according to any one of claims 1-5, wherein the detection reagent is a dye, such as a visible light dye or a fluorescent dye, such as an azo dye or an analog thereof (such as Congo red or Evans blue), a benzothiazole dye or an analog thereof (such as Thioflavin T and Thioflavin S), Amaranth red, Brilliant black, or Nile red.

7. The method according to any one of claims 1-6, wherein the separation matrix comprises one or more materials selected from cotton or cotton gauze; silk; a cellulose, such as nitrocellulose, microcrystalline cellulose, cellulose acetate, or wood chips; a polymer, such as polyester, polyethylene, polysulfone, polyvinyl alcohol, polyethylene glycol (such as PEG2000, PEG3000, PEG4000, PEG5000 or PEG6000), or polyacrylamide; glass fiber; silica gel, gelatin or dextran gel; a dry protein or a protein dry powder, such as egg white protein dry powder; an inorganic mineral soil, such as zeolite, clay, kaolin, hydroxyapatite and montmorillonite; a calcium salt, such as calcium chloride, calcium carbonate or calcium phosphate; activated carbon; or activated alumina.

8. An apparatus for detecting a misfolded protein or an aggregate thereof in a biological sample, wherein the apparatus comprises:
a housing defining a fluid separation chamber and a fluid collection chamber,
wherein, the fluid separation chamber is used to receive a mixed liquid of the biological sample and a detection reagent, wherein the detection reagent is capable of binding to the misfolded protein or the aggregate thereof in the biological sample, the fluid separation chamber contains a separation matrix constructed to adsorb the detection reagent that does not bind to the misfolded protein or the aggregate thereof from the mixed liquid and to allow passage of the detection reagent binding to the misfolded protein or the aggregate thereof; and
the fluid collection chamber is used to collect the biological sample flowing through the separation matrix in the fluid separation chamber.

9. The apparatus according to claim 8, wherein the detection reagent is a dye, such as a visible light dye or a fluorescent dye, such as an azo dye or an analog thereof (such as Congo red or Evans blue), a benzothiazole dye or an analog thereof (such as Thioflavin T and Thioflavin S), Amaranth red, Brilliant black, or Nile red.

10. The apparatus according to any one of claims 8-9, wherein the separation matrix comprises one or more materials selected from cotton or cotton gauze; silk; a cellulose, such as nitrocellulose, microcrystalline cellulose, cellulose acetate, or wood chips; a polymer, such as polyester, polyethylene, polysulfone, polyvinyl alcohol, polyethylene glycol (such as PEG2000, PEG3000, PEG4000, PEG5000 or PEG6000), or polyacrylamide; glass fiber; silica gel, gelatin or dextran gel; a dry protein or a protein dry powder, such as egg white protein dry powder; an inorganic mineral soil, such as zeolite, clay, kaolin, hydroxyapatite and montmorillonite; a calcium salt, such as calcium chloride, calcium carbonate or calcium phosphate; activated carbon; or activated alumina.

11. The apparatus according to any one of claims 8-10, wherein the fluid collection chamber is constructed to collect, in a free liquid state, the biological sample flowing through the fluid separation chamber.

12. The apparatus according to claim 11, wherein the collected biological sample can freely flow in the fluid collection chamber.

13. The apparatus according to claim 12, wherein the liquid that can freely flow in the fluid collection chamber has a volume of at least about 50 µL, at least about 100 µL, at least about 500 µL, at least about 1 mL, at least about 3 mL, or about 3 mL to about 5 mL.

14. The apparatus according to any one of claims 8-13, wherein the fluid collection chamber does not provide capillary action.

15. The apparatus according to any one of claims 8-14, wherein the apparatus further comprises an isolation layer disposed between the fluid separation chamber and the fluid collection chamber and separating the fluid separation chamber and the fluid collection chamber.

16. The apparatus according to claim 15, wherein the isolation layer allows the biological sample to flow through and prevents the separation matrix from entering the fluid collection chamber.

17. The apparatus according to claim 16, wherein the material of the isolation layer is selected from an inorganic material (such as an inorganic fiber or an inorganic particulate), such as a ceramic, a glass, a glass fiber, or a metal (such as stainless steel); a polymer, comprising a polymer fiber or a polymer particulate, such as a polyamide (such as nylon), a polyethylene (such as ultra-high molecular weight polyethylene (UHMW-PE), polytetrafluoroethylene (PTFE), polystyrene, or polyvinyl chloride (PVC)), a polyacrylic (such as acrylic), a polypropylene, a plastic (such as a porous plastic), a polyester and a polyurethane; a cellulose, such as filter paper or wood pulp cellulose, and optionally, the material is hydrophilic or has been subjected to a hydrophilic treatment.

18. The apparatus according to any one of claims 8-17, wherein the housing further defines a fluid receiving chamber for receiving the biological sample.

19. Use of the apparatus according to any one of claims 8-18 in the manufacture of a kit for diagnosing or predicting a disease **characterized by** a misfolded protein.
